# FASCICULE DE BREVET EUROPEEN

(11) **EP 3 157 946 B1**
(45) Date de publication et mention de la délivrance du brevet: **05.12.2018**
(21) Numéro de dépôt: 15738726.7
(22) Date de dépôt: 17.06.2015
(51) Int. Cl.: C12R 1/06, C07K 14/305, A61K 35/74, A61K 38/00, A23K 10/18, A23L 33/135, A23L 33/18, A23K 20/147

(54) **NOUVELLES SOUCHES D'ARTHROBACTER GANDAVENSIS**
ARTHROBACTER-GANDAVENSIS-STÄMME
ARTHROBACTER GANDAVENSIS STRAINS

(30) Priorité: 17.06.2014 FR 1455536
(43) Date de publication de la demande: 26.04.2017
(73) Titulaire: Adisseo France S.A.S., 92160 Antony (FR)
(72) Inventeur: PUJOL, Ange, F-13119 Saint Savournin (FR); FONS, Michel, F-13090 Aix en Provence (FR); MIRANDE, Caroline, F-01500 Ambérieu en Bugey (FR); DEVILLARD, Estelle, 03190 Vaux (FR); RHAYAT, Lamya, F-03430 Sauvagny (FR)
(74) Mandataire: Agasse, Stéphane
(86) Numéro de dépôt international: PCT/FR2015/051611
(87) Numéro de publication internationale: WO 2015/193618

(56) Documents cités:
- WO-A2-2006/104388
- WO-A2-2008/152252
- KR-A- 20080 075 389
- RIADH HAMMAMI ET AL: "Anti-infective properties of bacteriocins: an update", CELLULAR AND MOLECULAR LIFE SCIENCES, vol. 70, no. 16, août 2013 (2013-08), pages 2947-2967, XP055116653, ISSN: 1420-682X, DOI: 10.1007/s00018-012-1202-3
- M. WIETZ ET AL: "Wide Distribution of Closely Related, Antibiotic-Producing Arthrobacter Strains throughout the Arctic Ocean", APPLIED AND ENVIRONMENTAL MICROBIOLOGY, vol. 78, no. 6, 15 mars 2012 (2012-03-15), pages 2039-2042, XP055096498, ISSN: 0099-2240, DOI: 10.1128/AEM.07096-11
- KAMIGIRI K ET AL: "YM-30059, A NOVEL QUINOLONE ANTIBIOTIC PRODUCED BY ARTHROBACTER SP", JOURNAL OF ANTIBIOTICS, JAPAN ANTIBIOTICS RESEARCH ASSOCIATION, TOKYO, JP, vol. 49, no. 8, août 1996 (1996-08), pages 823-825, XP000974176, ISSN: 0021-8820

## Description

La présente invention a pour objet de nouvelles souches bactériennes ayant un intérêt en tant que probiotique dans l'alimentation animale et plus particulièrement pour l'alimentation des poulets.

Certaines souches bactériennes ont la capacité de libérer des substances ayant un effet bactériostatique ou bactéricide sur leurs compétiteurs. Ces substances antimicrobiennes peuvent être de nature organique, par exemple acides organiques ou peroxyde d'hydrogène (Ross et al., Int. J. Food Microbiol. 79, 3-16, 2002), ou de nature peptidique. On distingue, en outre, les peptides antimicrobiens synthétisés par voie enzymatique qui appartiennent à la classe des antibiotiques (Mootz et al., Curr. Opin. Chem. Biol. 1, 543-551, 1997; Keating et al., Curr. Opin. Chem. Biol. 3, 598-606, 1999), et les peptides produits par la voie ribosomique qui forment la classe des bactériocines (Jacob et al., Ann. Inst. Pasteur (Paris) 84, 222-224, 1953).

Les bactériocines suscitent un intérêt grandissant dans le monde de la recherche et de l'industrie; elles pourraient fournir des solutions alternatives à l'utilisation d'antibiotiques, notamment dans l'élevage (Luchansky, Antonie Van Leeuwenhoek 76, 335, 1999 ; O'Sullivan et al., Biochimie 84, 593-604, 2002).

De nombreux systèmes d'expression hétérologues de ces bactériocines se sont développés depuis quelques années. Notamment, Morisset et *al.* (Morisset et al., Appl. Environ. Microbiol., 70, 4672-4680, 2004) ont produit des variants de la mésentricine Y105, bactériocine de classe IIa produite par *Leuconostoc mesenteroides subsp*. *mesenteroides Y105,* chez *Leuconostoc mesenteroides subsp*. *dextranicum* DSM20484. De même, Flynn et al. (Microbiol., 148, 973-984, 2002) ont réalisé l'expression du gène de ABP-118, bactériocine de classe IIb produite à l'origine par *Lactobacillus salivarius subsp*. *salivarius* UCC118, chez les hôtes *Lactobacillus plantarum*, *Lactococcus lactis* et *Bacillus cereus.*

En outre, plusieurs essais ont été menés pour exprimer les gènes des bactériocines chez la bactérie *Escherichia coli* (McCormick et al., Appl. Environ. Microbiol., 64, 4757-4766, 1998; Garneau et al., Appl. Environ. Microbiol., 69, 1352-1358, 2003; Biet et al., Microbiol., 144, 2845-2854, 1998 ; Miller et al., Appl. Environ. Microbiol., 64, 14-20, 1998; Richard et al., J. Bacteriol., 186, 4276-4284, 2004; Kloche et al., Appl. Microbiol. Biotechnol., 67:532-538, 2005), la levure *Saccharomyces cerevisiae* (Schoeman et al., Yeast, 15, 647-656, 1999; Van Reenen et al., Int. J. Food Microbiol., 81, 29-40, 2003) et chez des bactéries lactiques (Rodriguez et al., Int. J. Food Microbiol., 80, 101-116, 2003).

De nombreux travaux sont donc menés en vue de l'identification de nouvelles bactériocines et de nouvelles souches bactériennes pour produire des bactériocines.

L'écosystème digestif est constitué par un microbiote abondant et très complexe regroupant des bactéries, des levures et des *Archaea.* Ce microbiote est essentiellement anaérobie et l'on retrouve principalement des bactéries des genres *Bacteroides*, *Eubacterium*, *Clostridium*, *Ruminococcus*, *Bifidobacterium* et *Fusobacterium* (Suau et al., Appl. Environ. Microbiol. 65, 4799-4807, 1999). Le microbiote a un impact important sur la santé de l'hôte. Il est notamment impliquée dans la toxification et la détoxication de composés métaboliques issus de l'alimentation (Hughes and Rowland, Microbial Ecology Health Disease 2, 179-185, 2000). Il est également capable de moduler l'expression de fonctions entérocytaires (Bry et al., Science 273,1380-1383, 1996 ; Hooper et al., Science 291, 881-884, 2001). Enfin, Il joue un rôle primordial dans la protection de l'hôte contre l'envahissement par des bactéries exogènes potentiellement pathogènes (Ducluzeau et al., Microbial Ecology and Intestinal Infections, 1988 ; Fons et al., Microbial Ecology in Health and Disease 2, 240-246, 2000).

Parmi les pathogènes intestinaux connus, on trouve *Clostridium perfringens,* bactérie à Gram positif, anaérobie stricte, capable de sporuler et très répandue dans l'environnement. Ce pathogène peut provenir de l'alimentation, mais peut aussi être présent à faible concentration dans l'intestin et se mettre à proliférer et sécréter des toxines sous l'effet d'un stress. Les souches de *Clostridium perfringens* sont classées en 5 toxinotypes en fonction des toxines qu'elles produisent (Petit et al., Trends Microbiol. 7, 104-110, 1999). Les souches de *C*. *perfringens* de type A sont responsables de maladies gastro-intestinales chez l'Homme. En 1997, plus de 245000 cas d'infections à *C*. *perfringens* ont été recensés aux Etats-Unis. Ceci a conduit à l'hospitalisation de 41 personnes dont 7 n'ont pas survécu (Mead et al., Emerg. Infect. Dis. 5, 607-625, 1999). Les souches de *C*. *perfringens* de type A et C peuvent être respectivement à l'origine d'entérite nécrotique chez les volailles et les porcs. Chez les volailles, l'entérite nécrotique est une pathologie aiguë, d'évolution rapide dont la mortalité peut atteindre 1 à 2% par jour. En plus de son incidence sur le bien-être des animaux, cette pathologie peut donc avoir une influence économique non négligeable. Jusqu'en 1999, cette maladie était bien contrôlée par l'usage d'antibiotiques comme facteurs de croissance. Mais, en 1999 l'Union européenne a interdit partiellement leur emploi, puis complètement en 2006 dans l'alimentation animale par crainte de sélectionner les bactéries résistantes et donc de voir diminuer l'efficacité des antibiotiques chez l'Homme. Depuis cette interdiction, l'entérite nécrotique provoquée par *Clostridium perfringens* chez la volaille et le porc, n'est plus contrôlée en Europe. Le nombre de cas déclarés au Réseau National d'Observations Epidémiologiques en Aviculture (RNOEA) (AFSSA Ploufragan) a considérablement augmenté en 1999 et 2000 (Valancony, Bulletin des GTV 12, 9-12, 2001).

Dabard et al. (Appl. Environ. Microbiol., 67, 4111-4118, 2001) ont montré que la souche *Ruminococcus gnavus* E1, isolée à partir de la flore dominante de l'Homme, est capable de produire une substance antimicrobienne, appelée ruminococcine A ou RumA, qui s'accumule dans le surnageant de culture. Il s'agit d'une bactériocine appartenant à la famille des lantibiotiques, active contre différentes souches de *Clostridium sp.* pathogènes. *Ruminococcus gnavus* est une bactérie anaérobie stricte appartenant à la famille des *Lachnospiraceae,* dans l'ordre des *Clostridiales.*

La demande de brevet WO 2008/152252 a pour objet une souche bactérienne de *Ruminococcus gnavus* (déposée à la CNCM sous le numéro I-3705 ainsi que les petides RumC1, RumC2 et RumC3 présentant une activité antibactérienne contre *Clostridium perfringens,* ainsi que les gènes codant pour ces peptides.

A ce jour, la recherche de solutions alternatives pour contrôler et traiter les maladies associées à la prolifération de *Clostridium perfringens* est donc de première importance.

La présente invention a permis d'identifier de façon tout à fait surprenante de nouvelles souches *d'Arthrobacter gandavensis* synthétisant des peptides ayant une activité antibactérienne (en tant que bactériocine) à l'encontre de *Clostridium perfringens.*

### Description de l'invention

La présente invention a pour objet une souche d'Arthrobacter gandavensis, ayant une activité contre *Clostridium perfringens* choisie parmi *Arthrobacter gandavensis* AP1 déposée le 19 février 2014 auprès de DSMZ sous le numéro DSM 28444, *Arthrobacter gandavensis* AP2 déposée le 19 février 2014 auprès de DSMZ sous le numéro DSM 28445, *Arthrobacter gandavensis* AP3 déposée le 19 février 2014 auprès de DSMZ sous le numéro DSM 28446, ou *Arthrobacter gandavensis* AP4 déposée le 19 février 2014 auprès de DSMZ sous le numéro DSM 28447.

Dans le cadre de la présente invention l'activité contre *Clostridium perfringens* peut être définie comme la capacité à inhiber la croissance ou le développement de bactéries cibles ou la capacité à tuer des bactéries cible. Les techniques de mesure de l'activité antimicrobienne sont connues de l'homme du métier. L'activité contre *Clostridium perfringens* peut être définie par un test d'activité tel que décrit au point 4.3 de l'exemple 4 ci-dessous ou dans la demande de brevet WO2008/152252 (et plus particulièrement à la page 23 & 24 : « 2. Test d'activité antimicrobienne à partir d'un échantillon liquide » ou page 24 : « 3. Test d'activité antimicrobienne à partir de colonies se développant en milieu gélosé » : L'activité antimicrobienne est dans ce cas mise en évidence dans la présente invention par un test d'inhibition de la souche *Clostridium perfringens* CpA mise en culture sur milieu gélosé. L'échantillon contenant l'un des peptides de l'invention est déposé dans des puits formés dans le milieu gélosé. L'activité antimicrobienne est mise en évidence lorsqu'est formé un halo d'inhibition autour du puit.

La présente divulgation a également pour objet un composé ayant une activité contre *C*. *perfringens* isolé à partir d'une souche batérienne choisie parmi *Arthrobacter gandavensis* AP1 déposée le 19 février 2014 auprès de DSMZ sous le numéro DSM 28444, *Arthrobacter gandavensis* AP2 déposée le 19 février 2014 auprès de DSMZ sous le numéro DSM 28445, *Arthrobacter gandavensis* AP3 déposée le 19 février 2014 auprès de DSMZ sous le numéro DSM 28446, ou *Arthrobacter gandavensis* AP4 déposée le 19 février 2014 auprès de DSMZ sous le numéro DSM 28447.

Dans un mode de réalisation particulier le peptide a une séquence choisie parmi SEQ ID No. 1 à SEQ ID No. 16.

La présente divulgation se rapporte également à des fragments biologiquement actifs de ces peptides ayant une activité antimicrobienne. Le terme "fragment biologiquement actifs " d'un peptide désigne un peptide comprenant une partie mais pas la totalité du peptide dont il est dérivé et qui a conservé l' activité antimicrobienne du polypeptide dont il est dérivé.

Les méthodes de préparation des peptides de séquences SEQ ID No. 1 à SEQ ID No. 16 sont connues de l'homme du métier.

Les séquences de ces peptides présentent de fortes identités avec les peptides RumC de la souche *Ruminococcus gnavus* déposée auprès de la CNCM sous le numéro I-3705. Les méthodes de mesure et d'identification du degré d'identité et du degré de similarité entre polypeptides sont connues de l'homme du métier. L'alignement des séquences est par exemple réalisé au moyen de Vector NTi 9.1.0, programme d'alignement AlignX (Clustal W algorithm) (Invitrogen INFORMAX, http://www.invitrogen.com) ou en utilisant l'outil CLUSTAW (http://www.ebi.ac.uk/clustalw/).

Les peptides tels que décrits ci-dessus sont secrétés (ou relargués) par les bactéries dans l'environnement extracellulaire. Il est possible que l'un quelconque des peptides de séquences SEQ ID No. 1 à SEQ ID No. 16 comprenne un peptide signal d'un nombre déterminé d'acides aminés. Dans ce cas, l'invention concerne également le peptide mature obtenu après clivage du peptide signal.

Dans un autre mode de réalisation, le peptide signal potentiel du peptide SEQ ID No. 1 à SEQ ID No. 16 peut être remplacé par un peptide signal hétérologue pour réaliser l'expression et la secrétion de ce peptide par un organisme hôte hétérologue.

Les peptides tels que décrits ci-dessus peuvent être isolés ou purifiés de leur environnement naturel. Ils peuvent notamment être isolés à partir de microbiotes caecaux et iléaux d'animaux et notamment de porcs hébergeant la souche *Arthrobacter gandavensis.* Les peptides peuvent être préparés au moyen de différents procédés. Ces procédés sont notamment la purification à partir de sources naturelles telles que des bactéries exprimant naturellement ces peptides, la production de peptides recombinants par des cellules hôtes appropriées et leur purification ultérieure, la production par synthèse chimique ou, enfin, une combinaison de ces différentes approches. Ainsi, les peptides des séquences SEQ ID No. 1 à 16 décrits ici peuvent être isolés à partir d'une des souches *d'Arthrobacter gandavensis* AP1 déposée auprès de DSMZ sous le numéro DSM 28444, *Arthrobacter gandavensis* AP2 déposée auprès de DSMZ sous le numéro DSM 28445, *Arthrobacter gandavensis* AP3 déposée auprès de DSMZ sous le numéro DSM 28446, ou *Arthrobacter gandavensis* AP4 déposée auprès de DSMZ sous le numéro DSM 28447.

Dans un autre mode de réalisation, les peptides tels que décrits ci-dessus sont isolés à partir d'organisme hôtes recombinants exprimant un composé ou un fragment d'un composé présentant une activité antimicrobienne.

Sont également divulguées des protéines de fusion, des protéines recombinantes ou des protéines chimères comprenant les peptides tels que décrits ci-dessus.

Selon un mode de réalisation le peptide est adapté à une utilisation en nutrition ou en pharmacie, par exemple une utilisation en nutrition animale.

On entend par "peptide adapté à l'utilisation dans la nutrition ou la pharmacie", un peptide dont les caractéristiques sont telles qu'il convient pour la nutrition ou la pharmacie. Les caractéristiques essentielles à une utilisation en nutrition ou en pharmacie sont notamment le pH auquel le peptide peut résister, la résistance aux enzymes gastriques et la conservation de leur activité à des températures physiologiques. En effet, une partie du système digestif des animaux et de l'homme est acide et il est donc essentiel que le peptide soit résistant à ce pH. Une autre caractéristique essentielle à une utilisation en nutrition est la température à laquelle la substance antimicrobienne est active. En effet, la mise en forme de substance antimicrobienne dans un médicament, un additif nutritionnel ou un aliment pour animaux, par exemple, implique des traitements et une température supérieure à la température ambiante. L'activité des antimicrobiens utilisés doit donc être stable dans les conditions des procédés, notamment les conditions de températures. Les antimicrobiens utilisés doivent également être actifs à des températures physiologiques (37-41°C).

Selon un mode de réalisation le peptide, ou un mélange de peptides selon l'invention, présente une activité antimicrobienne à pH neutre et conserve son activité antimicrobienne à un pH acide, par exemple inférieur à 7, de préférence inférieur à 4,4 et notamment à pH2.

Selon un mode de réalisation le peptide, ou un mélange de peptides selon l'invention, présente une activité antimicrobienne à 37°C et conserve cette activité à des températures inférieures et supérieures à la température ambiante, par exemple supérieure à 50°C.

Est également divulgué un polynucléotide codant pour un peptide ayant une activité contre *Clostridium perfringens* choisi parmi les polynucléotides dont la séquence est définie par SEQ ID No. 17 à SEQ ID No. 32, les polynucléotides qui s'hybrident au polynucléotide selon l'une quelconque des séquences SEQ ID No. 17 à SEQ ID No. 32, ou les polynucléotides codant pour un peptide tels que définis ci-dessus.

Selon la présente divulgation on entend par "polynucléotide" une chaîne nucléotidique simple brin ou son complémentaire pouvant être de type ADN ou ARN, ou une chaîne nucléotidique double brin pouvant être de type ADN complémentaire ou génomique. De préférence, les polynucléotides de l'invention sont de type ADN, notamment ADN double brin. Le terme "polynucléotide" désigne également les polynucléotides modifiés.

Les polynucléotides tels que décrits ci-dessus peuvent être isolés ou purifiés de leur environnement naturel. Les polynucléotides de la présente invention peuvent également être préparés par synthèse chimique ou par des techniques classiques de biologie moléculaire telles que décrites par Sambrook, Fristsch et Maniatis, dans leur ouvrage intitulé "Molecular cloning: a laboratory manual", édition : Cold Spring Harbor Laboratory Press, Cold Spring Harbor, NY, 1989.

Sont également divulgués des polynucléotides capables de s'hybrider de manière sélective avec le polynucléotide selon l'une quelconque des séquences SEQ ID No. 17 à SEQ ID No. 32

Dans le cadre de la présente divulgation une hybridation sélective est effectuée dans des conditions de moyenne stringence et préférentiellement dans des conditions de forte stringence. Par "séquence capable de s'hybrider de manière sélective", on entend les séquences qui s'hybrident avec la séquence de référence à un niveau supérieur au bruit de fond de manière significative. Le niveau du signal généré par l'interaction entre la séquence capable de s'hybrider de manière sélective et les séquences de référence est généralement 10 fois, de préférence 100 fois plus intense que celui de l'interaction des autres séquences d'ADN générant le bruit de fond. Les conditions d'hybridation stringentes permettant une hybridation sélective sont connues de l'homme du métier. En général, la température d'hybridation et de lavage est inférieure d'au moins 5°C au Tm de la séquence de référence à un pH donné et pour une force ionique donnée. Typiquement la température d'hybridation est d'au moins 30°C pour un polynucléotide de 15 à 50 nucléotides et d'au moins 60°C pour un polynucléotide de plus de 50 nucléotides. A titre d'exemple, l'hybridation est réalisée dans le tampon suivant : 6X SSC, 50 mM Tris-HCl (pH 7.5), 1 mM EDTA, 0.02% PVP, 0.02% Ficoll, 0.02% BSA, 500 µg/ml sperme de saumon denaturé DNA. Les lavages sont par exemple réalisés successivement à faible stringence dans un tampon 2X SSC, 0,1% SDS, à moyenne stringence dans un tampon 0,5X SSC, 01% SDS et à forte stringence dans un tampon 0,1X SSC, 0,1%SDS. L'hybridation peut bien entendu être effectuée selon d'autres méthodes usuelles connues de l'homme du métier (voir notamment Sambrook, Fristsch et Maniatis, dans leur ouvrage intitulé "Molecular cloning: a laboratory manual", édition : Cold Spring Harbor Laboratory Press, Cold Spring Harbor, NY, 1989). De préférence, les polynucléotides s'hybridant de manière sélective à un polynucléotide de référence conservent la fonction de la séquence de référence. Dans le cas présent, les polynucléotides s'hybridant de manière sélective avec le polynucléotide selon l'une quelconque des séquences SEQ ID No. 17 à SEQ ID No. 32. codent pour une activité anti-microbienne.

La divulgation se rapporte de manière générale aux polynucléotides codant pour les peptides tels que décrits ci-dessus. En raison de la dégénérescence du code génétique, différents polynucléotides peuvent coder pour un même polypeptide.

La présente divulgation concerne également une cassette d'expression caractérisée en ce qu'elle comprend dans le sens de la transcription un promoteur fonctionnel dans un organisme hôte, un polynucléotide tel que ci-dessus défini et une séquence terminatrice fonctionnelle dans ledit organisme hôte.

La présente divulgation concerne encore un vecteur comprenant un polynucléotide tel que ci-dessus défini et/ou une cassette d'expression telle que ci-dessus définie.

La présente divulgation concerne également des vecteurs de clonage ou d'expression pour la transformation d'un organisme hôte comprenant au moins un polynucléotide ou une cassette d'expression tels que décrits ci-dessus. Ce vecteur peut notamment correspondre à un plasmide, un cosmide, un bactériophage ou un virus dans lequel est inséré un polynucléotide ou une cassette d'expression selon l'invention. Les techniques de construction de ces vecteurs et d'insertion d'un polynucléotide de l'invention dans ces vecteurs sont connues de l'homme du métier. De manière générale, tout vecteur capable de se maintenir, de s'autorépliquer ou de se propager dans une cellule hôte afin d'induire notamment l'expression d'un polynucléotide ou d'un peptide peut être utilisé. L'homme du métier choisira les vecteurs appropriés en fonction de l'organisme hôte à transformer, et en fonction de la technique de transformation mise en oeuvre.

Les vecteurs de la présente divulgation sont notamment utilisés pour transformer un organisme hôte en vue de la réplication du vecteur et/ou de l'expression d'un peptide tel que décrit ci-dessus dans l'organisme hôte. La divulgation concerne aussi une méthode pour préparer un peptide tel que décrit ci-dessus comprenant les étapes suivantes:
- on transforme un organisme hôte avec un vecteur d'expression comprenant une cassette d'expression tels que décrits ci-dessus et/ou avec un polynucléotide tel que décrit ci-dessus
- on isole les peptides produits par l'organisme hôte.

La présente divulgation concerne aussi un organisme hôte transformé avec un polynucléotide tel que ci-dessus défini, une cassette d'expression telle que ci-dessus définie et/ou un vecteur tel que ci-dessus défini.

La présente divulgation a également pour objet, un procédé de transformation d'un organisme hôte par intégration dans ledit organisme hôte d'au moins un polynucléotide, d'au moins une cassette d'expression ou d'au moins un vecteur tels que décrits ci-dessus Le polynucléotide peut être intégré dans le génome de l'organisme hôte ou se repliquer de manière stable dans l'organisme hôte. Les méthodes de transformation des organismes hôtes sont connus de l'homme du métier et largement décrites dans la littérature.

Par "organisme hôte", on entend en particulier tout organisme mono ou pluricellulaire, inférieur ou supérieur, en particulier choisi parmi les bactéries, les levures et les champignons. En particulier, par "organisme hôte", on entend un organisme non humain. De manière avantageuse, les levures sont choisies parmi par exemple *Pichia pastoris, Saccharomyces cerevisae, Yarrowia lipolytica et Schwanniomyces occidentalis.* Les champignons sont par exemple choisis parmi les *Aspergillus,* les *Trichoderma* et les *Pénicilliums,* préférentiellement parmi *Penicillium funiculosum, Trichoderma reesei, Aspergillus niger, Aspergillus awamori, Aspergillus kawachii et Trichoderma koningii.* Dans un mode de réalisation de l'invention, l'organisme hôte est une souche de *Penicillium funiculosum* dans laquelle on exprime ou sur-exprime un peptide selon l'invention. Dans un autre mode de réalisation, l'organisme hôte est une souche de *Debaryomyces castellii* dans laquelle on exprime ou sur-exprime un peptide selon l'invention. Dans un autre mode encore de réalisation, l'organisme hôte est une souche d'Entérobactérie ou Corynébactérie et plus particulièrement *Escherichia coli, Bacillus subtilis, Corynebacterium glutamicum*, dans laquelle on exprime ou sur-exprime un peptide tel que décrit ci-dessus.

Les techniques de construction de vecteurs, de transformation d'organismes hôtes et d'expression de protéines hétérologues dans ces organismes sont largement décrites dans la littérature notamment par Sambrook, Fristsch et Maniatis, dans l'ouvrage intitulé "Molecular cloning: a laboratory manual", édition : Cold Spring Harbor Laboratory Press, Cold Spring Harbor, NY, 1989 ou par Ausubel et al., dans l'ouvrage intitulé "Current Protocols in Molecular Biology", édition : Greene Publishing Associates, Inc., and John Wiley and Sons, NY, 1992.

La divulgation concerne aussi un procédé de préparation d'un peptide présentant une activité antimicrobienne tel que décrit ci-dessus ledit procédé comprenant les étapes suivantes :
- mise en culture d'une souche de *Arthrobacter gandavensis* selon l'invention ou d'un organisme hôte transformé tel que décrit ci-dessus dans des conditions d'induction de l'expression du peptide, et
- récupération du surnageant de culture (ou moût de fermentation) comprenant le peptide.

La séparation du peptide à partir du surnageant de culture peut être réalisée par la charge, la taille et/ou le caractère hydrophobe. L'homme du métier connaît les différentes techniques permettant la séparation en fonction de la charge, de la taille et/ou du caractère hydrophobe des différents constituants d'un milieu.

Ce surnageant de culture ou moût de fermentation peut ensuite être concentré ou lyophilisé pour la formulation d'un additif alimentaire ou d'un aliment pour animaux. Le procédé peut comprendre des étapes supplémentaires de purification de la substance antimicrobienne à partir du surnageant de culture.

Si l'organisme hôte ne secrète pas la substance antimicrobienne dans le milieu de culture, une étape supplémentaire de casse des cellules et de purification de l'extrait cellulaire peut être nécessaire.

La présente invention concerne également une composition comprenant un moût de fermentation d'une souche selon l'invention. Selon un mode de réalisation de la présente invention, la composition se présente sous forme liquide ou sous forme de poudre.

Ces compositions comprennent différents ingrédients. Sous forme liquide, elles peuvent comprendre par exemple un autre agent antimicrobien, par exemple de l'acide sorbique ou un sel d'acide sorbique, de l'acide benzoïque ou un sel d'acide benzoïque, de l'acide fumarique ou un sel d'acide fumarique. Les compositions telles que décrites ci-dessus peuvent en outre comprendre du sorbitol. Le sorbitol est un agent de stabilisation et de formulation. Les compositions telles que décrites ci-dessus peuvent également comprendre des agents anticongelants, par exemple l'éthylène glycol, le glycérol, le propylène glycol et le propane-1,2-diol.

Les compositions telles que décrites ci-dessus comprennent au moins un peptide tel que décrit ci-dessus mais elles peuvent également comprendre d'autres substances comme des vitamines, des principes actifs autres, des acides aminés ou des sels minéraux.

Les compositions sous forme de poudre comprennent un support. Ce support peut être choisi parmi la farine de blé, l'amidon, la maltodextrine, le gypsum et les rafles de maïs.

Les compositions selon l'invention présentent une activité antimicrobienne. Elles fournissent des solutions alternatives à l'utilisation d'antibiotiques. Elles peuvent par exemple être utilisées dans l'élevage des animaux ou comme médicament pour l'homme.

La présente invention concerne également un additif nutritionnel comprenant une souche selon l'invention ou un moût de fermentation d'une souche selon l'invention. Selon un mode de réalisation de la présente invention, l'additif se présente sous forme liquide ou sous forme de poudre.

La présente invention concerne aussi un aliment pour animaux caractérisé en ce qu'il comprend une base nutritionnelle pour animaux et un additif nutritionnel tel que ci-dessus défini.

Ces aliments se présentent habituellement sous la forme de farines ou de granulés dans lesquels sont incorporées les compositions présentant une activité antimicrobienne.

Dans le cadre de la présente invention, on entend par "aliment" tout ce qui peut servir à la nourriture des animaux. Par "base nutritionnelle", on entend tout ce qui constitue l'essentiel de la ration alimentaire de l'animal, constituée à titre d'exemple par un mélange de céréales, de protéines et de matières grasses d'origine animale et/ou végétale. Habituellement, ces bases nutritionnelles comprennent par exemple du maïs, du blé, et du soja. Ces bases nutritionnelles sont adaptées aux besoins des différentes espèces animales auxquelles elles sont destinées. Il peut par exemple s'agir de volailles (poules pondeuses, poulets de chair, dindes et canards) ou de porcs (porcs croissance et finition, porcelets, truies).

L'invention a pour objet l'utilisation d' au moins un mout de fermentation d'une souche selon l'invention et/ou au moins d'une souche selon l'invention pour la préparation d'un additif nutritionnel, d'un aliment ou d'un médicament.

Le peptide tel que ci-dessus défini, le mout de fermentation d'une souche ou d'un organisme hôte tel que ci-dessus défini, la souche ou l'organisme hôte tel que décrit ci-dessus peuvent être utilisés comme médicament.

Le peptide tel que défini ci-dessus, le mout de fermentation d'une souche ou d'un organisme hôte tel que ci-dessus défini, la souche ou l'organisme hôte tel que décrit ci-dessus peuvent être utilisés prévenir ou traiter les maladies gastro-intestinales chez l'homme.

De façon particulière, le peptide tel que décrit ci-dessus, le mout de fermentation d'une souche ou d'un organisme hôte tel que ci-dessus défini, la souche ou l'organisme hôte tel que décrit ci-dessus peuvent être utilisés pour la prévention et/ou le traitement de dysbactérioses intestinales, notamment l'entérite nécrotique chez les animaux monogastriques, et plus particulièrement les volailles et les porcs

La présente invention a également pour objet l'utilisation d' au moins un mout de fermentation d'une souche selon l'invention et/ou d'au moins additif nutritionnel selon l'invention ou d'un aliment selon l'invention pour améliorer les performances de croissance des animaux, notamment du poulet.

La présente invention a également pour objet l'utilisation d' au moins un mout de fermentation d'une souche selon l'invention et/ou au moins d'une souche selon l'invention et/ou d'un additif nutritionnel selon l'invention et/ou d'un aliment selon l'invention pour améliorer les performances zootechniques des animaux d'élevage.

Dans le cadre de la présente invention l'amélioration des performances zootechniques des animaux d'élevage comprend de façon non exhaustive l'augmentation du gain de poids des animaux, la diminution de l'indice de consommation, la diminution de la mortalité et morbidité, l'homogénéité des animaux, l'amélioration rendement carcasse/rendement viande, l'amélioration de la digestibilité des nutriments, l'amélioration du statut immunitaire de l'animal, la réduction des effets négatifs d'une infection à pathogène (*Clostridium perfingens*, *Clostridium difficile, E. coli*, *Salmonella sp., Campylobacter sp*.), ou encore l'amélioration de l'utilisation des nutriments et donc réduction de l'excrétion de rejets.

### Description des figures

**Figure 1** : Détection du gène *rumC1* par PCR
**Figure 2** **:** Profil chromosomique par électrophorèse en champ pulsé des souches AP1, AP2, AP3 et AP4.
**Figure 3** : Pourcentage de survie dans la nourriture des souches AP1, AP2, AP3 et AP4

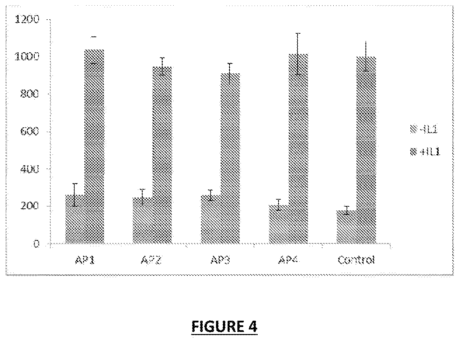

**Figure 4** : dosage des interleukines IL8 par ELISA dans le surnageant de culture des cellules Caco-2 après contact avec les bactéries en présence ou en absence d'IL1
**Figure 5** : Les tests d'activité avec les surnageants de culture bactérien contre la souche *Clostridium perfringens*
**Figure 6** **:** une activité anti-C. *perfringens* après une première étape de pré-purification a été réalisée : purification des surnageants sur colonne Sep-Pak avec une élution à 40% d'acétonitrile (ACN, cf. WO2008/152252)

La présente invention sera illustrée par les exemples suivants

### Exemple 1 : Isolement de souches bactériennes rumC+

La recherche de souches cultivables hébergeant les gènes *rumC-*like a été entreprise à partir des microbiotes caecaux et iléaux de porcs.

Dans un premier temps les bactéries sont mises en culture dans les milieux suivants :
- M17 : favorisant les lactocoques
- LB : permettant la croissance des *Bacillus* sp. et entérocoques
- BEA : milieu contre-sélectionnant le groupe *Bacteroides*

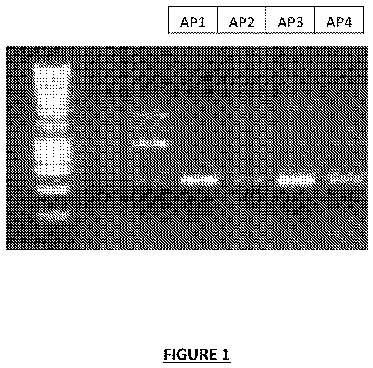

Les clones sont ensuite sélectionnés pour leur capacité à inhiber la croissance de *Clostridium perfringens.* La présence des gènes *rumC* est alors mise en évidence par PCR (Figure 1).

**Tableau 1 : Liste des amorces utilisées pour amplifier les différents fragments d'ADN cibles**

| Couple d'amorces | Température d'hybridation (Tₒₚₜ) | Taille du fragment amplifié | Gène(s) ciblé(s) |
|---|---|---|---|
| FC1 (SEQ ID No.33) - RC1(SEQ ID No.34) | 55°C | 700 pb | *rumC1* |
| FC2C3(SEQ ID No.35) -RC2C3(SEQ ID No.36) | 60°C | 800 pb | *rumC2-rumC3* |
| FC4C5(SEQ ID No.37) -RC4C5(SEQ ID No.38) | 55°C | 400 pb | *rumC4-rumC5* |

Quatre souches ont été retenues : AP1, AP2 AP3 et AP4.

### Exemple 2 : Identification des souches retenues

### 2.1 ADNr 16S

Un fragment (environ 1550 pb) du gène codant l'ARNr 16S (correspondant aux positions 8-1541 dans le système de numérotation *d'Escherichia coli*) a été amplifié par PCR en utilisant des amorces conservées (16F8 : 5'-AGAGTTTGATCCTGGCTGAG-3' (SEQ ID No.39) et 16R1541 : 5'-AAGGAGGTGATCCAGCCGCA-3') (SEQ ID No.40) puis séquencé.

Les séquences obtenues ont été soumises à une comparaison dans les banques de données à l'aide d'un programme de recherche d'homologie de séquence de type « BLAST »
Souche AP1 (SEQ ID No.) => 99,45% d'identité avec *Arthrobacter gandavensis* R 5812
Souche AP2 (SEQ ID No.) => 99,37% d'identité avec *Arthrobacter gandavensis* R 5812
Souche AP3 (SEQ ID No.) => 99,44% d'identité avec *Arthrobacter gandavensis* R 5812
Souche AP4 (SEQ ID No.) => 99,31% d'identité avec *Arthrobacter gandavensis* R 5812

### 2.2. Identification PFGE

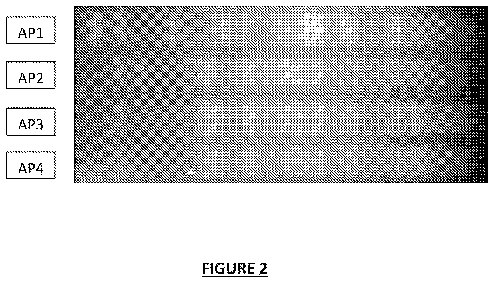

Les souches AP1, AP2 AP3 et AP4 appartenant au genre *Arthrobacter* doivent pouvoir être différenciées sur le plan génétique. La technique de référence pour identifier au niveau intra-spécifique des souches bactériennes consiste à établir leur profil chromosomique par électrophorèse en champ pulsé (Figure 2) (Rapport d'analyse N°: LR251012 - Biocéane).

Les souches AP3 et AP4 semblent identiques. Cependant, le séquençage des gènes rumC, ainsi que leurs séquences peptidiques réduites, semble quant à lui indiquer que AP3 et AP4 sont deux souches distinctes (cf. Exemple 4, point1 4.2).

### Exemple 3 : Caractérisation des souches

### 3.1 Résistance au pH et aux sels biliaires

Les souches sont soumises à deux traitements pour déterminer leur résistance à l'acidité et aux sels biliaires.
- Acidité : tampon NaCl 0.85%, pH2, contenant de la pepsine (1mg/mL)
- Sels Biliaire tampon isotonique (K₂HPO₄ 1.24%, H₂PO₄ 0.76%, citrate trisodique 0.1%, [NH₄]₂SO₄ 0.6%, pH6.7) contenant 0.2% de sels biliaire (50% cholate de sodium, 50% desoxycholate de sodium)

**Tableau 2 : Pourcentage de survie des différentes souches**

| | AP1 | AP2 | AP3 | AP4 |
|---|---|---|---|---|
| Acidité | 0% | 15% | 12% | 90% |
| SB | 0% | 10% | 8% | ND |

| | | | | |
|---|---|---|---|---|
| SB : Sels biliaires ; ND : Non déterminé | | | | |

La souche AP4 résiste le mieux aux conditions mimant le milieu gastrique. De façon générale, toutes les souches sont plus sensibles aux sels biliaires mais la survie est cependant suffisante, excepté pour la souche AP1.

### 3.2 Paramètres fermentaires

L'analyse de leurs paramètres fermentaires a été réalisée sur un surnageant de culture obtenue après croissance en BHI-YH en semi-anaérobiose (analyse © In vivo Labs).

**Tableau 3 : Dosage des paramètres fermentaires**

| | AP1 | AP2 | AP3 | AP4 | BHI-YH |
|---|---|---|---|---|---|
| Acide lactique % | 0,01 | 0,01 | 0,01 | 0,02 | 0,04 |
| Azote ammoniacal g/L | <0,05 | <0,05 | <0,05 | <0,05 | <0,05 |
| Acide fumarique % | <0,01 | <0,01 | <0,01 | <0,01 | <0,01 |
| Acide acétique g/L | 0,16 | 0,14 | 0,13 | 0,15 | 0,15 |
| Acide propionique g/L | <0,1 | <0,1 | <0,1 | <0,1 | <0,1 |
| Acide isobutyrique g/L | <0,1 | <0,1 | <0,1 | <0,1 | <0,1 |
| Acide butyrique g/L | <0,1 | <0,1 | <0,1 | <0,1 | <0,1 |
| Acide isovalérique g/L | <0,1 | <0,1 | <0,1 | <0,1 | <0,1 |
| Acide valérique g/L | <0,1 | <0,1 | <0,1 | <0,1 | <0,1 |

Comme attendu, il est difficile d'évaluer les paramètres fermentaires pour les souches *d'Arthrobacter*. Les conditions de culture ne permettent pas de mettre en évidence la production d'un quelconque métabolite issu de la fermentation. Il a également été recherché la présence d'acide propionique, d'acide isobutyrique, d'acide butyrique, d'acide isovalérique et d'acide valérique. Cependant aucunes des quatre souches ne semblent être productrice dans nos conditions de culture.

### 3.3 Tests de survie à la température

Les souches sont sensibles aux températures élevées. En effet, aucune ne survit au-delà de 70°C.

Selon ces résultats, il est difficilement envisageable de prévoir un ajout de ces souches lors de la granulation.

### 3.4 Tests de survie dans l'eau et les aliments

La souche *Arthrobacter* sp. AP4 survit très bien dans l'eau (Tableau 4 ci-dessous). Il semblerait même qu'elle soit capable de se développer dans ces conditions. En effet, après 7 jours dans l'eau, la population bactérienne aurait doublé.

**Tableau 4 : Pourcentage de survie dans l'eau**

| | J1 | J2 | J3 | J4 | J7 |
|---|---|---|---|---|---|
| AP4 | 134% | 144% | 130% | 166% | 206% |

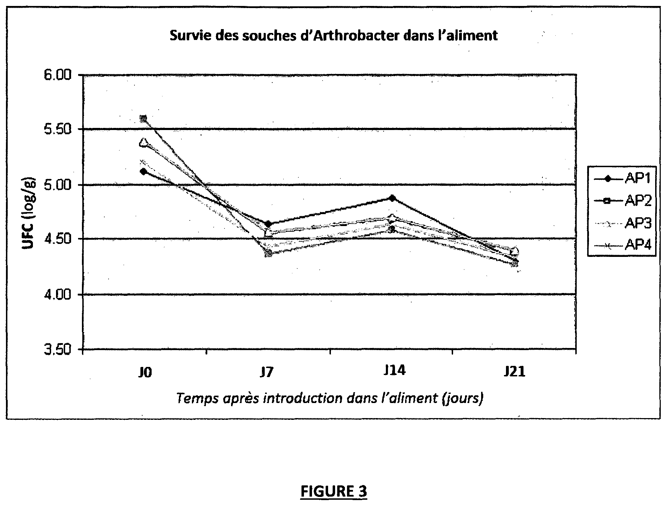

Dans l'aliment la population reste relativement stable même après 21 jours (Figure 3). Selon ces résultats, l'addition des souches aussi bien dans l'eau de boisson que dans l'aliment est envisageable.

### 3.5 Potentiel anti-inflammatoire

La modulation du profil inflammatoire est estimé par dosage des interleukines IL8 par ELISA dans le surnageant de culture des cellules Caco-2 (lignée cellulaire intestinale) après contact avec les bactéries en présence ou en absence d'IL1 (molécule d'induction de l'inflammation).

Ces résultats ont été obtenus sur les surnageants de cellules cultivées en puits. Les bactéries ne présente pas d'activité pro-inflammatoire (sécrétion d'IL8 faible en absence d'IL1) ni d'activité anti-inflammatoire (quantité d'IL8 en présence d'IL1 identique au control).

L'expérience a été répétée avec la souche AP4 mais avec des cellules Caco-2 cultivées sur filtre. Dans ce cas cette souche présente une activité pro-inflammatoire.

### 3.6 Profils enzymatiques

Le système API ZYM est une méthode semi quantitative de recherche d'activités enzymatiques. Les tests enzymatiques sont inoculés avec une suspension bactérienne dense.

**Tableau 5 : Résultats des lectures des galeries Api Zym**

| | AP1 | | AP2 | | AP3 | | AP4 | |
|---|---|---|---|---|---|---|---|---|
| | I | II | I | II | I | II | I | II |
| 1. Témoin sans substrat | - | - | - | - | - | - | - | - |
| 2. Phosphatase alcaline | - | - | - | - | - | - | - | - |
| 3. Estérase (C4) | 5 | 5 | 5 | 4 | 5 | 4 | 5 | 5 |
| 4. Estérase lipase (C8) | 1 | 3 | 4 | 4 | 3 | 4 | 3 | 5 |
| 5. Lipase (C14) | - | - | - | - | - | - | - | - |
| 6. Leucine arylamidase | 5 | 5 | 5 | 5 | 5 | 5 | 5 | 5 |
| 7. Valine arylamidase | 3 | 1 | 4 | 1 | 4 | 1 | 4 | 1 |
| 8. Cystine arylamidase | 3 | - | 4 | - | 4 | - | 4 | - |
| 9. Trypsine | - | - | - | - | - | - | - | - |
| 10. Alpha-Chymotrypsine | - | - | - | - | - | - | - | - |
| 11. Phosphatase acide | 3 | 3 | 4 | 3 | 4 | 2 | 4 | 3 |
| 12. Naphtol phosphohydrolase | - | 1 | - | 1 | - | 1 | - | 1 |
| 13. Alpha-galactosidase (mélibiase) | - | - | - | - | - | - | - | - |
| 14. Béta-galactosidase (lactase) | - | - | - | - | - | - | - | - |
| 15. Béta-glucuronidase (hyaluronidase) | - | - | - | - | - | - | - | - |
| 16. Alpha-glucosidase (maltase) | 2 | 1 | - | - | - | - | - | - |
| 17. Béta-glucosidase (cellulase) | - | - | - | - | - | - | - | - |
| 18. N-acétyl-béta-glucosaminidase (chitinase) | - | - | - | - | - | - | - | - |
| 19. Alpha-mannosidase | - | - | - | - | - | - | - | - |
| 20. Alpha-fucosidase | - | 1 | - | - | - | - | - | - |

| | | | | | | | | |
|---|---|---|---|---|---|---|---|---|
| I : premier essai ; II : second essai | | | | | | | | |

Certaines activités sont retrouvées indiscutablement quelle que soit la souche utilisée. C'est le cas de l'estérase, de l'estérase lipase, de la leucine arylamidase, de la valine arylamidase et de la phosphatase acide. Certaines activités ne semblent pas très stables, comme pour la cystine arylamidase.

La souche AP1 présente un profil différent des autres *Arthrobacter* dans l'utilisation du maltose et du fucose par exemple.

### 3.7 Résistance aux antibiotiques

Un premier essai a été réalisé au laboratoire. Les antibiogrammes ont été réalisés en utilisant des disques de diffusion antibiotique (BBL™ Sensi-Disc™ Susceptibility Test Dises).

Les antibiotiques testés ont été utilisés aux quantités suivantes : Bacitracine 10µg, érythromycine 15µg, pénicilline G 10µg, ampicilline 10µg, vancomycine 30µg, streptomycine 300µg, chloramphénicol 30µg, ciprofloxacine 5µg, fosfomycine 200µg, rifampicine 25µg et triméthoprime / sulfaméthoxazole 1,25µg / 23,75µg. Les résultats de l'antibiogramme sont soumis à un abaque de lecture afin de déterminer le niveau de sensibilité de la souche par rapport au diamètre d'inhibition mesuré.

Les 4 souches *d'Arthrobacter* sont sensibles à tous les antibiotiques testés.

**Tableau 6 : sensibilité aux antibiotiques**

| | **AP1** | **AP2** | **AP3** | **AP4** |
|---|---|---|---|---|
| **Ampicilline** | S | S | S | S |
| **Erythromycine** | S | S | S | S |
| **Gentamicine** | S | S | S | S |
| **Kanamycine** | S | S | S | S |
| **Streptomycine** | **R** | **R** | **R** | **R** |
| **Tetracycline** | **R** | S | S | S |
| **Chloramphenicol** | **R/S** | **R/S** | S | S |
| **Vancomycine** | S | S | S | S |
| **Ciprofloxacine** | ND* | ND* | ND* | ND* |
| **Linezolid** | ND* | ND* | ND* | ND* |
| **Clindamycine** | **R** | S | S | S |
| **Tylosine** | ND* | ND* | ND* | ND* |

| | | | | |
|---|---|---|---|---|
| ND* : non déterminé car absence de break-point | | | | |

D'après ces tests toutes les souches sont résistantes à la Streptomycine. La souche AP1 est celle qui présente le plus de résistances.

### 3.8 Tests d'adhésion

L'adhésion bactérienne est estimée sur des cellules Caco-2 (lignée cellulaire épithéliale).

**Tableau 7 : Numération bactérienne et taux d'adhésion**

| Souches | CFU_{I} | CFU_{A} |
|---|---|---|
| AP1 | 4,90.10⁸ | 1,50.10³ |
| AP2 | 1,50.10⁹ | 3,00.10³ |
| AP3 | 1,75.10⁵ | 6,00.10³ |
| AP4 | 1,42.10⁹ | 3,00.10⁴ |

| | | |
|---|---|---|
| CFU_{I} : dénombrement initial CFU_{A} : dénombrement après adhésion | | |

Bien que faible, toutes nos souches présentent une capacité d'adhésion aux cellules intestinales. Les souches AP3 et AP4 semblent adhérer plus efficacement.

### Exemple 4 : Validation du concept

### 4.1 Innocuité des souches

Dans un premier temps les bactéries ont été observées en microscopie électronique afin de vérifier l'absence de caractère morphologique associé à la pathogénicité.

La morphologie des cellules est variable (bâtonnets à coccoïdes), ce qui est en accord avec la caractérisation du genre *Arthrobacter.* Les cellules sont dépourvues de flagelles et de pili.

Dans un second temps un test *in vivo* a été mis en place. 10⁷ bactéries ont été administrées en intra-gastrique à des souris axéniques (3 animaux par souche). Chaque jour et pendant 5 jours un prélèvement de fèces est effectué. Une analyse de ces selles par microscopie optique a permis de confirmer la présence des bactéries pendant au moins 4 jours, mettant en évidence leur survie dans le tube digestif. Il est à noter une absence de mortalité, de lésions intestinales et de signes cliniques (prostration, diarrhées...). Ces résultats appuient l'expérimentation sur la lignée cellulaire Caco-2 (Absence de lyse ou de décollement des cellules).

### 4.2 Séquençage des gènes rumC

Les séquences des différents gènes rumC-like présents chez nos souches ont été comparées aux séquences de la souche *R. gnavus* E1 (Cf Annexes).

**Tableau 8 : Pourcentage d'identité des gènes rumC-like par rapport aux gènes de R. gnavus E1 (les identifiants de séquences figurant dans le tableau ci-dessous correspondent aux séquences identifiées dans les souches AP1 à AP4).**

| | **AP1** | **AP2** | **AP3** | **AP4** |
|---|---|---|---|---|
| ***rumC1*** | 78,12 (SEQ ID No 17) | 99,48 (SEQ ID No 22) | 93,23 (SEQ ID No 27) | 98,44 (SEQ ID No 30) |
| ***rumC2*** | 97,92 (SEQ ID No 18) | 55,73 (SEQ ID No 23) | 86,46 (SEQ ID No 28) | 84,9 (SEQ ID No 31) |
| ***rumC3*** | 100 (SEQ ID No 19) | 44,79 (SEQ ID No 24) | 89,06 (SEQ ID No 29) | 89,06 (SEQ ID No 32) |
| ***rumC4*** | 60,98 (SEQ ID No 20) | 75,14 (SEQ ID No 25) | / | / |
| ***rumC5*** | 52,2 (SEQ ID No 21) | 66,88 (SEQ ID No 26) | / | / |

La conservation des gènes est différente en fonction des souches. Le gène *rumC1* est celui qui est le plus conservé. De façon général les gènes *rumC4* et *rumC5* sont très divergents, voire trop divergents, pour être séquencés pour les souches AP3 et AP4.

La même analyse a été effectuée en comparant les séquences peptidiques déduites (Cf. annexes).

**Tableau 9 : Pourcentage d'identité des séquences peptidiques déduites par rapport à R. gnavus E1 (les identifiants de séquences figurant dans le tableau ci-dessous correspondent aux séquences identifiées dans les souches AP1 à AP4).**

| | **AP1** | **AP2** | **AP3** | **AP4** |
|---|---|---|---|---|
| **RumC1** | 55,56 (SEQ ID No 1) | 98,41 (SEQ ID No 6) | 62,3 (SEQ ID No 11) | 75,81 (SEQ ID No 14) |
| **RumC2** | 79,37 (SEQ ID No 2) | 25,4 (SEQ ID No 7) | 77,78 (SEQ ID No 12) | 76,19 (SEQ ID No 15) |
| **RumC3** | 100 (SEQ ID No 3) | 3,17 (SEQ ID No 8) | 92,06 (SEQ ID No 13) | 92,06 (SEQ ID No 16) |
| **RumC4** | 7,69 (SEQ ID No 4) | 14,29 (SEQ ID No 9) | | |
| **Rumc5** | 15,38 (SEQ ID No 5) | 33,96 (SEQ ID No 10) | | |

Globalement les mêmes conclusions peuvent être tirées. Les identités des séquences déduites sont inférieures aux identités des gènes. Dans certains cas, les séquences peptidiques sont trop éloignées (ou tronquées ; Cf annexes) pour prétendre à une activité (RumC2_AP3 et RumC4_AP1 par exemple).

### 4.3 Test d'activité

Les tests d'activité sont réalisés avec les surnageants de culture bactérien contre la souche *Clostridium perfringens.*

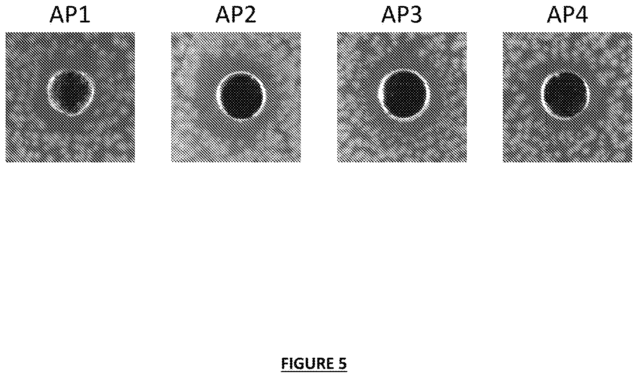

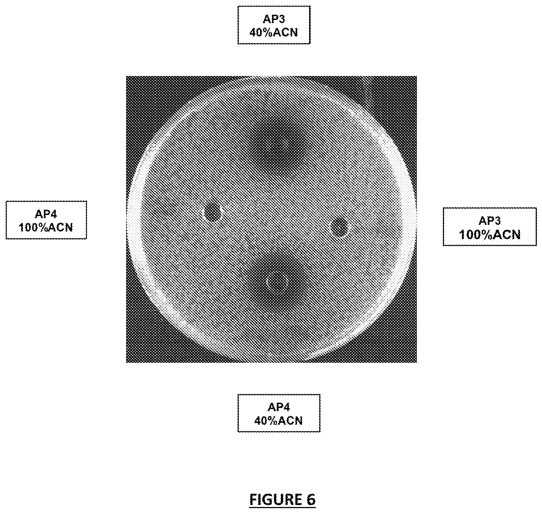

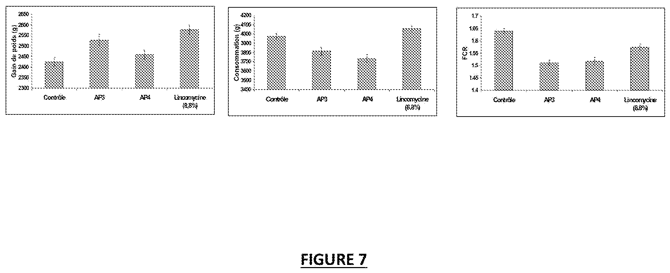

Les quatre souches possèdent bien une activité anti-C. *perfringens* (Figure 5). Afin de limiter l'action d'un acide organique les surnageants sont neutralisés avant le test. Deux tests ont été réalisés afin de conforter l'idée que l'inhibition de *C*. *perfringens* est bien due à une activité « RumC-like ». Dans un premier temps les surnageants ont été chauffés 10 min à 95°C avant le test. L'activité est bien conservée comme c'est le cas pour RumC. Dans un second temps la première étape de pré-purification a été réalisée (cf. WO 2008/152252): purification des surnageants sur colonne Sep-Pak avec une élution à 40% d'acétonitrile (ACN, Figure 6). Dans ce cas l'activité est également retrouvée.

### 4.4 Evaluation des propriétés probiotiques des souches d'Arthrobacter in vivo

L'évaluation de l'effet des souches AP3 et AP4 sur les performances de croissance du poulet de chair (gain de poids, consommation et indice de consommation) a été réalisée dans des conditions dites de "régime challengeant". Ce régime consiste en un régime à base de maïs avec une teneur standard en protéines (23%) durant 14 jours suivi d'un régime à base de blé et d'orge, donc riche en fibres, hyperprotéiné (26% de protéines) de 14 à 35 jours. Ce régime challengent correspond au « régime « contrôle du tableau 10).

Les souches AP3 et AP4 ont été pulvérisées sur l'aliment à une concentration permettant l'ingestion de 10⁸ CFU par jour et par animal, dès le premier jour, et ce, pendant toute la durée de l'essai, à savoir 35 jours. Ces régimes correspondent respectivement aux mentions « AP3 » et « AP4 » dans le tableau 10.

Le régime « Lincomycine (8,8%) » correspond à un régime challengeant auquel est ajouté de la lycomycine à hauteur de 8,8% (i.e. 5,25g par tonne d'aliment).

Ces quatre traitement ont été effectués sur des lots de 15 poulets et ont été répétés 12 fois (soit 720 poulets au total).

### Tableau 10 : résultats in vivo.

**ET** : Ecart-Type; **IC** : Indice de consommation (consommation d'aliment nécessaire à l'augmentation du gain de poids de 1kg, IC = Consommation/Gain de Poids) ; % : pourcentage d'amélioration par rapport au contrôle; **Consommation** : consommation d'aliment par les animaux sur la totalité de l'essai

| | **Gain de poids** (g) | ET | % | **Consommation** (g) | ET | % | **IC** | ET | % | Mortalité |
|---|---|---|---|---|---|---|---|---|---|---|
| **Contrôle** | **2425** | 21.4 | | **3974** | 28.0 | | **1.639** | 0.009 | | 3.3% |
| **AP3** | **2527** | 28.2 | **4.2%** | **3815** | 42.6 | **-4.0%** | **1.51** | 0.012 | **-7.9%** | 3.3% |
| **AP4** | **2460** | 21.5 | **1.4%** | **3733** | 43.3 | **-6.1%** | **1.518** | 0.015 | **-7.4%** | 1.1% |
| **Lincomycine (8,8%)** | **2577** | 22.4 | **6.3%** | **4057** | 37.7 | **2.1%** | **1.575** | 0.013 | **-3.9%** | 1.7% |

Les deux souches testées, AP3 et AP4, ont des effets similaires et positifs sur les performances de croissance des poulets de chair. Elles permettent une diminution de l'indice de consommation de 7 à 8% due à la fois à une augmentation du gain de poids et à une diminution de la consommation

## Revendications

1. Souche *d'Arthrobacter gandavensis* ayant une activité contre *Clostridium perfringens* choisie parmi les souches AP1 déposée auprès de DSMZ le 19 février 2014 sous le numéro DSM 28444, AP2 déposée auprès de DSMZ le 19 février 2014 sous le numéro DSM 28445, AP3 déposée auprès de DSMZ le 19 février 2014 sous le numéro DSM 28446 ou AP4 déposée auprès de DSMZ le 19 février 2014 sous le numéro DSM 28447.

2. Composition comprenant au moins un mout de fermentation d'une souche selon la revendication 1, ladite composition étant de préférence sous la forme d'un liquide ou d'une poudre.

3. Additif nutritionnel comprenant au moins une souche *d'Arthrobacter gandavensis* selon la revendication 1, et/ou au moins un mout de fermentation d'une souche selon la revendication 1, ledit additif étant de préférence sous la forme d'un liquide ou d'une poudre.

4. Aliment pour animaux **caractérisé en ce qu'**il comprend un additif nutritionnel selon la revendication 3 et une base nutritionnelle.

5. Utilisation d'au moins un mout de fermentation d'une souche selon la revendication 1 et/ou au moins une souche selon la revendication 1 pour la préparation d'un additif nutritionnel, d'un aliment ou d'un médicament.

6. Mout de fermentation d'une souche selon la revendication 1, ou souche selon la revendication 1 pour son utilisation comme médicament.

7. Mout de fermentation d'une souche selon la revendication 1, ou souche selon la revendication 1 pour son utilisation dans le traitement et/ou la prévention desdysbactérioses intestinales et plus particulièrement de l'entérite nécrotique chez les animaux monogastriques et plus particulièrement les volailles et les porcs.

8. Utilisation d'au moins une souche selon la revendication 1, d'au moins un mout de fermentation d'une souche selon la revendication 1 et/ou d'au moins un additif nutritionnel selon l'une des revendications 3 et/ou d'au moins un aliment selon la revendication 4 pour améliorer les performances de croissance des animaux, d'élevage notamment du poulet ou du porc.

9. Utilisation d'au moins une souche selon la revendication 1, et/ou d'au moins un mout de fermentation d'une souche selon la revendication 1 et/ou d'au moins un additif nutritionnel selon l'une des revendications 3 et/ou d'au moins un aliment selon la revendication 4 pour améliorer les performances zootechniques des animaux d'élevage.

## Patentansprüche

1. Stamm von *Arthrobacter gandavensis* mit einer Aktivität gegen *Clostridium perfringens*, ausgewählt aus den Stämmen AP1, hinterlegt bei der DSMZ am 19. Februar 2014 unter der Nummer DSM 28444, AP2, hinterlegt bei der DSMZ am 19. Februar 2014 unter der Nummer DSM 28445, AP3, hinterlegt bei der DSMZ am 19. Februar 2014 unter der Nummer DSM 28446, oder AP4, hinterlegt bei der DSMZ am 19. Februar 2014 unter der Nummer DSM 28447.

2. Zusammensetzung, umfassend mindestens einen Fermentierungsmost eines Stamms nach Anspruch 1, wobei die Zusammensetzung vorzugsweise die Form einer Flüssigkeit oder eines Pulvers aufweist.

3. Nahrungsmittelzusatz, umfassend mindestens einen Stamm von *Arthrobacter gandavensis* nach Anspruch 1 und/oder mindestens einen Fermentierungsmost eines Stamms nach Anspruch 1, wobei der Zusatz vorzugsweise die Form einer Flüssigkeit oder eines Pulvers aufweist.

4. Tierfutter, **dadurch gekennzeichnet, dass** es einen Nahrungsmittelzusatz nach Anspruch 3 und eine Nahrungsmittelbasis umfasst.

5. Verwendung von mindestens einem Fermentierungsmost eines Stamms nach Anspruch 1, und/oder mindestens einem Stamm nach Anspruch 1 zur Herstellung eines Nahrungsmittelzusatzes, eines Futters oder eines Arzneimittels.

6. Fermentierungsmost eines Stamms nach Anspruch 1, oder Stamm nach Anspruch 1, für ihre Verwendung als Arzneimittel.

7. Fermentierungsmost eines Stamms nach Anspruch 1, oder Stamm nach Anspruch 1, für seine Verwendung bei der Behandlung und/oder Prävention der intestinalen Dysbakteriose und insbesondere der nekrotisierenden Enteritis bei monogastrischen Tieren und insbesondere Geflügel und Schweinen.

8. Verwendung von mindestens einem Stamm nach Anspruch 1, mindestens einem Fermentierungsmost eines Stamms nach Anspruch 1, und/oder mindestens einem Nahrungsmittelzusatz nach einem der Ansprüche 3 und/oder mindestens ein Futter nach Anspruch 4, um die Wachstumsleistungen der Zuchttiere, insbesondere des Geflügels oder der Schweine, zu verbessern.

9. Verwendung von mindestens einem Stamm nach Anspruch 1, und/oder mindestens einem Fermentierungsmost eines Stamms nach Anspruch 1 und/oder mindestens einem Nahrungsmittelzusatz nach einem der Ansprüche 3 und/oder mindestens einem Futter nach Anspruch 4, um die zootechnischen Leistungen der Zuchttiere zu verbessern.

## Claims

1. An *Arthrobacter gandavensis* strain having an activity against *Clostridium perfringens* selected from the strains AP1 deposited at DSMZ on February 19, 2014 under the number DSM 28444, AP2 deposited at DSMZ on February 19, 2014 under the number DSM 28445, AP3 deposited at DSMZ on February 19, 2014 under the number DSM 28446 or AP4 deposited at DSMZ on February 19, 2014 under the number DSM 28447.

2. A composition comprising at least one fermentation must of a strain according to claim 1, said composition being preferably in the form of a liquid or a powder.

3. A nutritional additive comprising at least one *Arthrobacter gandavensis* strain according to claim 1, and/or at least one fermentation must of a strain according to claim 1, said additive being preferably in the form of a liquid or a powder.

4. An animal feed **characterized in that** it comprises a nutritional additive according to claim 3 and a nutritional base.

5. A use of at least one fermentation must of a strain according to claim 1 and/or at least one strain according to claim 1 for the preparation of a nutritional additive, a feed or a drug.

6. A fermentation must of a strain according to claim 1, or a strain according to claim 1 for its use as a drug.

7. A fermentation must of a strain according to claim 1, or a strain according to claim 1 for its use in the treatment and/or the prevention of intestinal dysbacterioses and more particularly of necrotic enteritis in monogastric animals and more particularly poultry and pigs.

8. A use of at least one strain according to claim 1, at least one fermentation must of a strain according to claim 1 and/or of at least one nutritional additive according to any of claims 3 and/or at least one feed according to claim 4 for improving the performances of growth of breeding animals in particular chicken or pork.

9. A use of at least one strain according to claim 1 and/or at least one fermentation must of a strain according to claim 1 and/or at least one nutritional additive according to any of claims 3 and/or at least one feed according to claim 4 for improving the zootechnical performances of breeding animals.


### SEQUENCE LISTING

<110> ADISSEO FRANCE SAS
<120> Nouvelles souches d'Arthrobacter gandavensis
<130> BR079715
<160> 40
<170> PatentIn version 3.5
<210> 1
   <211> 63
   <212> PRT
   <213> Arthorbacter gandavensis
<400> 1
   Met Asn Ile Ile Ala Thr Tyr Gln Ser Leu Lys Glu Asn Gln Asn Gln
   1               5                   10                  15
   Asp Tyr Thr Pro Arg Leu Phe Val Phe Ala Gly Lys Ala Val Ala Asn
               20                  25                  30
   Ser His Asn Ala Gly Pro Ala Tyr Cys Val Gly Tyr Cys Gly Asn Asn
           35                  40                  45
   Gly Val Val Thr Arg Asn Ala Asn Ala Asn Val Ala Lys Thr Ala
       50                  55                  60
<210> 2
   <211> 64
   <212> PRT
   <213> Arthorbacter gandavensis
<400> 2
   Met Arg Lys Ile Val Ala Gly Lys Leu Gln Thr Gly Ala Asp Phe Glu
   1               5                   10                  15
   Gly Ser Lys Gly Gly Cys Lys Cys Ser Gly Gly Ala Val Val Glu Asn
               20                  25                  30
   Ser His Asn Ala Gly Pro Ala Tyr Cys Val Gly Tyr Cys Gly Asn Asn
           35                  40                  45
   Gly Gly Asp Asn Lys Lys Cys Glu Cys Tyr Ser Cys Lys Asn Lys Ile
       50                  55                  60
<210> 3
   <211> 63
   <212> PRT
   <213> Arthorbacter gandavensis
<400> 3
   Met Lys Leu Val Glu Thr Lys Thr Thr Lys Thr Gly Thr Asn Phe Glu
   1               5                   10                  15
   Gly Asn Arg Ala Gly Cys Ile Cys Asn Gly Thr Val Ala Val Ala Asn
               20                  25                  30
   Ser His Asn Ala Gly Pro Ala Tyr Cys Val Gly Tyr Cys Gly Asn Ser
           35                  40                  45
   Gly Val Val Thr Arg Asn Ala Asn Ala Asn Val Ala Lys Thr Ala
       50                  55                  60
<210> 4
   <211> 52
   <212> PRT
   <213> Arthorbacter gandavensis
<400> 4
   Trp Lys Leu Asn Met Ser Asp Thr His His Phe Glu Glu Gly Lys Pro
   1               5                   10                  15
   Gly Arg Gly Gly Arg Val Thr Asn Arg Gly Glu Thr Ile Ile Met Val
               20                  25                  30
   Phe Arg Cys Trp Val Glu Cys Gly Pro Ser Asn Tyr Gln Lys Ser Ile
           35                  40                  45
   Asn Asn Tyr Glu
       50
<210> 5
   <211> 52
   <212> PRT
   <213> Arthorbacter gandavensis
<400> 5
   Lys Asn Cys Glu Arg Thr Leu Ile Ser Lys Glu Arg Leu Asp Val Leu
   1               5                   10                  15
   Leu Ser Leu Phe Leu Arg Gly Val Cys Arg Asn Pro Asn Ser Asn Lys
               20                  25                  30
   Pro Gly Val Leu Val Val Trp Phe Trp Gly Glu Phe Gly Glu Pro Thr
           35                  40                  45
   Arg Asn Asp Met
       50
<210> 6
   <211> 64
   <212> PRT
   <213> Arthorbacter gandavensis
<400> 6
   Met Arg Lys Ile Val Ala Gly Lys Leu Gln Thr Gly Ala Asp Phe Glu
   1               5                   10                  15
   Gly Ser Lys Trp Gly Cys Val Cys Ser Gly Ser Thr Ala Val Ala Asn
               20                  25                  30
   Ser His Asn Ala Gly Pro Ala Tyr Cys Val Gly Tyr Cys Thr Arg Asn
           35                  40                  45
   Asn Gly Val Val Thr Arg Asn Ala Asn Ala Asn Val Ala Lys Thr Ala
       50                  55                  60
<210> 7
   <211> 65
   <212> PRT
   <213> Arthorbacter gandavensis
<400> 7
   Met Arg Ser Ile Glu Glu Cys Met Glu Thr Trp Ile Leu Glu Cys Arg
   1               5                   10                  15
   Gly Ser Gly Leu His Val Arg Asn Ala Lys Tyr Gly Arg Thr Pro Val
               20                  25                  30
   Ala Lys Ala Ala Val Gly Leu Tyr Arg Glu Ala Arg Lys Ser Gly Gln
           35                  40                  45
   Thr Gly Leu Asp Thr Val Gly Asn Ala Asn Ala Asn Leu Ala Arg Thr
       50                  55                  60
   Lys
   65
<210> 8
   <211> 63
   <212> PRT
   <213> Arthorbacter gandavensis
<400> 8
   Met Lys Leu Val Gly Gly Phe Pro Pro Gln Ala Ala Ala Ser Ile Lys
   1               5                   10                  15
   His Leu Gln Gly Ser Met Ala Ala Arg Thr Gln Arg Lys Arg Gly Leu
               20                  25                  30
   Leu Cys Gly Glu His Trp Ile Arg Pro Arg Arg Ser Phe Gln Leu Gln
           35                  40                  45
   Phe Gly Val Met Met Gln Ala Val Gln Lys Ser His Asn Gln Pro
       50                  55                  60
<210> 9
   <211> 56
   <212> PRT
   <213> Arthorbacter gandavensis
<400> 9
   Val Arg Glu Ala Leu His Ile Ala Ser Thr Phe Arg Arg Thr Gln Ala
   1               5                   10                  15
   Val Phe Ala Ala Asp Cys Arg Leu Glu Ile Leu Ile Met Gln Asp Gln
               20                  25                  30
   His Ile Val Leu Gly Met Val Glu Arg Trp Ala Val Thr Arg Asn Gln
           35                  40                  45
   Asn Tyr Thr Leu Glu Gln Arg Ser
       50                  55
<210> 10
   <211> 53
   <212> PRT
   <213> Arthorbacter gandavensis
<400> 10
   Leu Gly Thr Ala Lys Pro Ile Lys Thr Gln Asn Asn Phe Glu Gly Asn
   1               5                   10                  15
   Gly Ala Gly Cys Phe Cys Gly Gly Ser Val Ala Ile Gln Ile Leu Ile
               20                  25                  30
   Ser Arg Glu Phe Leu Leu Cys Gly Phe Gly Gly Asn Leu Gly Asn Gln
           35                  40                  45
   Pro Ala Met Ile Cys
       50
<210> 11
   <211> 61
   <212> PRT
   <213> Arthorbacter gandavensis
<400> 11
   Met Arg Lys Ile Val Ala Gly Lys Leu Gln Thr Gly Ala Asp Phe Glu
   1               5                   10                  15
   Gly Ser Lys Trp Trp Met Met Gln Trp Arg Cys Ser Ser Arg Asn Ser
               20                  25                  30
   His Asn Ala Gly Pro Ala Tyr Cys Val Gly Tyr Cys Gly Asn Asn Gly
           35                  40                  45
   Gly Leu Glu Met Leu Met Gln Met Ser Gln Lys Arg His
       50                  55                  60
<210> 12
   <211> 63
   <212> PRT
   <213> Arthorbacter gandavensis
<400> 12
   Met Arg Lys Ile Val Ala Gly Lys Leu Gln Thr Gly Ala Asp Phe Glu
   1               5                   10                  15
   Gly Ser Arg Ser Gly Cys Val Cys Ser Gly Ser Ala Ala Val Ala Ser
               20                  25                  30
   Ser His Tyr Ala Gly Pro Ala Tyr Cys Val Gly Tyr Cys Gly Asn His
           35                  40                  45
   Gly Ala Val Thr Arg Asn Ala Asn Tyr Asn Leu Ala Lys Lys Asn
       50                  55                  60
<210> 13
   <211> 63
   <212> PRT
   <213> Arthorbacter gandavensis
<400> 13
   Met Lys Leu Val Glu Thr Lys Thr Thr Lys Thr Gly Thr Asp Phe Glu
   1               5                   10                  15
   Gly Asn Arg Ala Gly Cys Ile Cys Asn Gly Thr Val Ala Val Ala Asn
               20                  25                  30
   Ser His Tyr Ala Gly Pro Ala Tyr Cys Val Gly Tyr Cys Gly Asn Ser
           35                  40                  45
   Gly Val Val Thr Arg Asn Ala Asn Ala Asp Leu Ala Arg Thr Ala
       50                  55                  60
<210> 14
   <211> 62
   <212> PRT
   <213> Arthorbacter gandavensis
<400> 14
   Met Ser Asn Ile Leu Ala Gly Lys Leu Gln Thr Gly Ala Asp Phe Glu
   1 5 10 15
   Gly Ser Lys Trp Gly Cys Val Cys Ser Gly Ser Thr Ala Val Ala Asn
   20 25 30
   Ser His Asn Ala Gly Pro Ala Tyr Cys Val Gly Tyr Cys Gly Asn Asn
   35 40 45
   Gly Val Ser Asp Lys Cys Cys Lys Cys Arg Lys Asn Gly Asn
   50 55 60
<210> 15
   <211> 63
   <212> PRT
   <213> Arthorbacter gandavensis
<400> 15
   Met Arg Lys Ile Val Ala Gly Lys Leu Gln Thr Gly Ala Asp Phe Glu
   1 5 10 15
   Gly Ser Arg Ser Gly Cys Val Cys Ser Ala Ser Ala Ala Val Ala Ser
   20 25 30
   Ser His Tyr Ala Gly Pro Ala Tyr Cys Val Gly Tyr Cys Gly Asn Arg
   35 40 45
   Gly Ala Val Thr Arg Asn Ala Asn Tyr Asn Leu Ala Lys Lys Asn
   50 55 60
<210> 16
   <211> 63
   <212> PRT
   <213> Arthorbacter gandavensis
<400> 16
   Met Lys Leu Val Glu Thr Lys Thr Thr Lys Thr Gly Thr Asp Phe Glu
   1 5 10 15
   Gly Asn Arg Ala Gly Cys Ile Cys Asn Gly Thr Val Ala Val Ala Asn
   20 25 30
   Ser His Tyr Ala Gly Pro Ala Tyr Cys Val Gly Tyr Cys Gly Asn Ser
   35 40 45
   Gly Val Val Thr Arg Asn Ala Asn Ala Asp Leu Ala Arg Thr Ala
   50 55 60
<210> 17
   <211> 192
   <212> DNA
   <213> Arthorbacter gandavensis
<400> 17
   ttgaacatta ttgccactta tcaatcattg aaagagaacc agaaccaaga ctacacacca        60
   cgcttgttcg tctttgccgg taaagcagta gcaaactctc ataatgcagg accggcgtat       120
   tgcgtaggat actgtggaaa caacggagta gtgactagaa atgctaatgc aaatgtcgca       180
   aaaacggcat aa                                                           192
<210> 18
   <211> 193
   <212> DNA
   <213> Arthorbacter gandavensis
<400> 18
   atgagaaaaa tcgtagcagg aaagttacag acaggagcag actttgaagg cagcaaaggt        60
   ggatgtaaat gcagtggcgg tgcagtagta gaaaactctc ataatgcagg accagcgtat       120
   tgcgtgggat actgtggaaa caacggagga gataacaaga aatgcgaatg ctattcttgc       180
   aagaacaaaa taa                                                          193
<210> 19
   <211> 192
   <212> DNA
   <213> Arthorbacter gandavensis
<400> 19
   atgaaattag tagaaacaaa aacaacaaaa acaggaacaa actttgaagg gaatagagct        60
   ggatgtattt gtaatggcac tgtagcagta gcaaattctc ataatgcagg accagcatat       120
   tgtgttgggt attgcggaaa tagtggagta gtaacaagaa atgcgaatgc aaatgtcgca       180
   aaaacagcat aa                                                           192
<210> 20
   <211> 164
   <212> DNA
   <213> Arthorbacter gandavensis
<400> 20
   tggaagctaa attagatgag cgacacacat cactttgaag aaggtaagcc aggccggggt        60
   ggccgcgtaa caaacagagg cgaaactatt ataatggtgt ttagatgttg ggtggagtgt       120
   gggccctctt agaactatca aaaatcaatt aataactatg aaag                        164
<210> 21
   <211> 159
   <212> DNA
   <213> Arthorbacter gandavensis
<400> 21
   caaaaattgt gagagaacac ttatttcaaa ggagagactg gatgtactgc tgtcgctgtt 60
   tttgcggggg gtctgtcgca atccaaattc taataagccg ggagttcttg ttgtgtggtt 120
   ttggggggaa tttggggaac caacccgcaa tgatatgtt 159
<210> 22
   <211> 198
   <212> DNA
   <213> Arthorbacter gandavensis
<400> 22
   atgagaaaaa tcgtagcagg aaagttacag acaggagcag actttgaagg cagcaaatgg 60
   ggatgtgttt gtagtggaag cacagcagta gcaaactctc ataatgcagg accggcgtat 120
   tgcgtaggat actgtacata gagaaacaac ggagtagtga ctagaaatgc taatgcaaat 180
   gtcgcaaaaa cggcataa 198
<210> 23
   <211> 216
   <212> DNA
   <213> Arthorbacter gandavensis
<400> 23
   atgaggtcaa tcgaagaatg catggaaact tggatacttg agtgctgaag aggcagtgga 60
   ctccatgtgt agcggtgaaa tgcgtagaaa tatggaagaa ccccagtggc gaaggcggca 120
   gtcggtctgt actgacgtga ggctcgaaag tctgggtagc aaacaggatt agatacggta 180
   ggaaatgcga atgcgaatct tgcaagaaca aaataa 216
<210> 24
   <211> 210
   <212> DNA
   <213> Arthorbacter gandavensis
<400> 24
   atgaaattag ttggagggtt tccgcctcaa gcggccgcta gcattaagca tcttcagggg 60
   agtatggccg caaggtgaac tcaaaggaag cgggggcttc tctgcggaga gcattggtaa 120
   atttgaagac cgcgaaggtc cttccagctg cagtttgggg tgatgtaaat gcaggcggtg 180
   cagtagtaga aatctcataa tcagccataa 210
<210> 25
   <211> 173
   <212> DNA
   <213> Arthorbacter gandavensis
<400> 25
   gggtacgcga agcactgcac atcgcttcaa cttttaggag gacgcaggct gtgtttgcag 60
   cggactgtcg acttgaaatt ctcataatgc aggaccagca tattgtgttg gggatggtgg 120
   aaagatgggc agtaaccaga aatcaaaatt atactcttga acaaaggtct taa 173
<210> 26
   <211> 160
   <212> DNA
   <213> Arthorbacter gandavensis
<400> 26
   ttaggaacag caaaaccaat aaaaacgcaa aataattttg aaggaaacgg ggctggttgt 60
   ttttgcgggg ggtctgtcgc aatccaaatt ctaataagcc gggagttctt gttgtgtggt 120
   tttgggggga atttggggaa ccaacccgca atgatatgtt 160
<210> 27
   <211> 194
   <212> DNA
   <213> Arthorbacter gandavensis
<400> 27
   atgagaaaaa tcgtagcagg aaagttacag acaggagcag actttgaagg cagcaaatgg 60
   tggatgtaaa tgcagtggcg gtgcagtagt agaaactctc ataatgcagg tccagcgtat 120
   tgcgtgggat actgtggaaa caacggaggg tagtgactag aaatgctaat gcaaatgtcg 180
   caaaaacggc ataa 194
<210> 28
   <211> 192
   <212> DNA
   <213> Arthorbacter gandavensis
<400> 28
   atgagaaaaa tcgtagcagg aaagttacag acaggagcag actttgaagg cagcagaagt 60
   ggatgtgttt gtagtggaag cgcagcagta gcaagctctc attatgcagg accagcatat 120
   tgcgtaggat actgtggaaa ccatggagca gtaacaagaa atgcgaatta taatcttgca 180
   aaaaaaaact ag 192
<210> 29
   <211> 192
   <212> DNA
   <213> Arthorbacter gandavensis
<400> 29
   atgaaattag tagaaacaaa aacaacaaaa acaggaacag actttgaagg aaatagggct 60
   ggatgtattt gtaatggtac tgtagcagtg gcaaattctc attatgcagg accggcatat 120
   tgtgtgggat attgtggcaa cagtggagtg gtgacaagaa atgccaatgc agatcttgca 180
   agaactgcat ag 192
<210> 30
   <211> 194
   <212> DNA
   <213> Arthorbacter gandavensis
<400> 30
   atgagcaata tcttagcagg aaagttacag acaggagcag actttgaagg cagcaaatgg 60
   ggatgtgttt gtagtggaag cacagcagta gcaaactctc ataatgcagg accggcgtat 120
   tgcgtaggat actgtggaaa caacggagta agtgactaga aatgctaatg caaatgtcgc 180
   aaaaacggca ataa 194
<210> 31
   <211> 192
   <212> DNA
   <213> Arthorbacter gandavensis
<400> 31
   atgagaaaaa tcgtagcagg aaagttacag acaggagcag actttgaagg cagcagaagt 60
   ggatgtgttt gtagtgcaag cgcagcagta gcaagctctc attatgcagg accagcatat 120
   tgcgtaggat actgtggaaa ccgtggagca gtaacgagaa atgcgaatta taatcttgca 180
   aaaaaaaact ag 192
<210> 32
   <211> 192
   <212> DNA
   <213> Arthorbacter gandavensis
<400> 32
   atgaaattag tagaaacaaa aacaacaaaa acaggaacag actttgaagg aaatagggct 60
   ggatgtattt gtaatggtac tgtagcagtg gcaaattctc attatgcagg accggcatat 120
   tgtgtgggat attgtggcaa cagtggagtg gtgacaagaa atgccaatgc agatcttgca 180
   agaactgcat ag 192
<210> 33
   <211> 20
   <212> DNA
   <213> Artificial Sequence
<220>
   <223> Primer
<400> 33
   tggggatgtg tttgtagtag 20
<210> 34
   <211> 20
   <212> DNA
   <213> Artificial Sequence
<220>
   <223> Primer
<400> 34
   tccgtttttg cgacatttgc 20
<210> 35
   <211> 30
   <212> DNA
   <213> Artificial Sequence
<220>
   <223> Primer
<400> 35
   atgaatgctg ggcgagaaat ttgtgcggac 30
<210> 36
   <211> 30
   <212> DNA
   <213> Artificial Sequence
<220>
   <223> Primer
<400> 36
   ggagaccgca cctactggac aatctccttc 30
<210> 37
   <211> 22
   <212> DNA
   <213> Artificial Sequence
<220>
   <223> Primer
<400> 37
   gaaaggagga acaaaacatg ag 22
<210> 38
   <211> 20
   <212> DNA
   <213> Artificial Sequence
<220>
   <223> Primer
<400> 38
   tcgtgccaga ttagcatttg 20
<210> 39
   <211> 20
   <212> DNA
   <213> Artificial Sequence
<220>
   <223> Primer
<400> 39
   agagtttgat cctggctgag 20
<210> 40
   <211> 20
   <212> DNA
   <213> Artificial Sequence
<220>
   <223> Primer
<400> 40
   aaggaggtga tccagccgca 20